# EUROPEAN PATENT SPECIFICATION

(11) **EP 1 998 690 B1**
(45) Date of publication and mention of the grant of the patent: **31.07.2019**
(21) Application number: 07748342.8
(22) Date of filing: 16.03.2007
(51) Int. Cl.: A61B 17/11, A61F 2/04, A61L 27/50

(54) **A METHOD FOR MANUFACTURING A NERVE REGENERATION DEVICE**
VERFAHREN ZUR HERSTELLUNG EINER NERVENREGENERATIONSVORRICHTUNG
PROCÉDÉ POUR FABRIQUER UN DISPOSITIF DE RÉGÉNÉRATION DE NERFS

(30) Priority: 29.03.2006 SE 0600708
(43) Date of publication of application: 10.12.2008
(73) Proprietor: Swenora Biotech AB, 181 63 Lidingö (SE)
(72) Inventor: SVENSSON, Mikael, S-113 40 Stockholm (SE); MATTSSON, Per, S-183 77 Täby (SE)
(74) Representative: AWA Sweden AB
(86) International application number: PCT/SE2007/050166
(87) International publication number: WO 2007/111562

(56) References cited:
- EP-A1- 1 586 285
- WO-A1-88/06871
- WO-A1-90/05552
- WO-A1-98/04197
- WO-A2-00/66036
- DE-A1- 10 309 237
- US-A- 5 925 053
- US-A- 6 090 117
- US-A1- 2004 102 793
- US-B1- 6 214 021

## Description

### Technical Field

The present invention uses a mould by which a device for nerve regeneration in the spinal cord can be manufactured. Said device can be used to treat a spinal cord injury. The invention is a method of manufacturing such a device.

### Background and Related Art

In contrast to the peripheral nervous system, the central nervous system (CNS) is unable to heal when injured. At present an injury to the spinal cord in humans leads to permanent paralysis below the place of the injury.

Several attempts have been made to overcome these problems and enable regrowth of axons in the spinal cord. Both growth inhibiting factors and growth enhancing factors have been identified. Growth enhancing factors such as Brain-derived neurotrophic factor (BDNF), glial-derived neurotrophic factor (GDNF and acidic fibroblast neurotrophic factor (aFGF) have all proved to be successful in improving the function after a spinal cord injury. Chondroitinase ABC (Ch ABC) has been shown to reduce the effect of inhibiting chondroitine sulphate, hence enabling regeneration of central axons.

The nerve paths of a healthy person run in the white matter of the spinal cord. It is known that injured axons in the CNS will grow into grafts of peripheral nerves. Attempts have been made to bridge the injured area by using several peripheral nerve grafts to extend the central axons to the caudal part of the spinal cord. Such methods are based on the idea that the white matter of the spinal cord comprises the inhibiting factors, whereas the grey substance is more permissive to regrowth. At present, therefore the peripheral nerve grafts may be arranged so that the newly grown axons will direct signals from the white matter to the grey matter of the spinal cord, as a strategy to circumvent the inhibitory environment for nerve regeneration in the white matter. Detailed knowledge is available about the location of the white and grey matter, respectively, in the human spinal cord, and about the positions of the axons.

The redirection from white matter to grey matter will create axons that may significantly improve, although not fully restore, the patient's function.

Cheng, H., Y. Cao and L. Olson: "Spinal cord repair in adult paraplegic rats: partial restoration of hind limb function", Science, 1996, 274 (5274): p. 510-3 discloses such a method. According to this article, peripheral nerves taken from another part of the animal's body are manually placed in such a way that they bridge the injury, from the white matter to the grey matter. The method is difficult and imprecise. Despite many attempts this method has been difficult to reproduce elsewhere.

International Patent Application No. WO98/04197 proposes the use of a device having a substantially cylindrical form and containing holes or channels for bridging openings in the proximal end with openings in the distal end. The channels may be filled with peripheral nerves taken from the patient. When the device is placed in the injured part of the spinal cord, the peripheral nerves will grow together with the central axons of the spinal cord, thus interconnecting the proximal and the distal end of the injured area.

A problem associated with connecting white matter to grey matter is that the connections will not follow straight lines. When several nerve grafts are to be applied, the nerve regeneration device must be made in such a way that these nerve grafts do not intercept each other and that they are positioned exactly where they should be anatomically in order to restore function to the greatest degree possible.

For producing the device, WO 98/04197 briefly proposes the use of flexible tubes around which the biocompatible material is moulded, to achieve the desired channels.

US 6,214,021 B1 discloses a method of manufacturing a nerve regeneration device comprising providing a mould comprising a bottom plate, a top plate and a centre part, each of said plates having a number of holes, and said centre part having a bore therethrough, placing wires in the bore, each exiting the mould through one hole in the top plate and a hole in the bottom plate, filling the mould with a material to produce the device, removing the device from the mould and removing the wires from the device.

DE 103 09 237 A1 discloses a method of manufacturing a nerve regeneration device in which a spinal cord injury is imaged, and a natural sponge grown or shaped on the basis of the image data forms the nerve regeneration device, in which the sponge's breathing canals constitute open channels.

### Object of the Invention

It is an object of the invention to provide a method and mould for manufacturing a nerve regeneration device for treating spinal cord injuries, that can be individually adapted.

### Summary of the Invention

This object is achieved according to the present invention by a method of manufacturing a nerve regeneration device for treating a spinal cord injury of a patient, said method comprising the following steps:
- providing a mould comprising a base plate, a top plate and a centre part, each of said base plate and said top plate having a number of holes corresponding to points in the injured spinal cord where nerves should be regenerated, and said centre part having a channel therethrough,
- placing first flexible elongate structures in the channels, each first flexible elongate structure exiting the mould through one hole in the top plate and a matching hole in the base plate,
- filling the mould with a biocompatible or biodegradable material to produce the device,
- removing the device from the mould when ready;
- removing the first flexible elongate structure from the device.

In conjunction with this method, or at a later stage, the following step may be performed, together or at separate points in time:
- inserting nerves, and/or biological material promoting nerve regeneration, in the channels.

A mould for manufacturing a device for treating a spinal cord injury of a patient, said mould comprising a base plate, a top plate and a centre part, each of said base plate and said top plate having a number of holes corresponding to points in the injured spinal cord where nerves should be regenerated, and said centre part having a channel therethrough, arranged to receive a number of threads, each thread exiting the mould through one hole in the top plate and a matching hole in the base plate. Using the method and the mould according to the invention, a nerve regeneration device specifically adapted to an individual injury in the spinal cord can be produced, which will allow axons in the damaged area to regenerate and restore function. The resulting device can be adapted to a specific wound in order to ascertain an exact and reproducible anatomical positioning of the channels.

The method comprises the preparatory steps of imaging the spinal cord injury using an imaging technique, obtaining image data related to the shape and size of the injury and providing the mould having a shape and size matching the spinal cord injury on the basis of said image data. This enables the manufacturing individually adapted nerve regeneration devices for a particular patient or injury. The imaging technique may be a radiological imaging technique, such as computer tomography (CT) or magnetic resonance imaging (MRI) or any other imaging technique used in the treatment of patients.

The biocompatible material or biodegradable material may comprise an agent to stimulate nerve regeneration. This agent will then be distributed gradually to the regenerating nerves in order to stimulate growth.

Preferably, each of the first structures is placed through holes in the top plates situated to correspond to grey matter area and holes in the base plates situated to correspond to white matter area, and vice versa. This is feasible because nerve regeneration is more readily achieved in grey matter than in white matter.

The step of inserting nerves in the channels may comprise the steps of inserting second threads in the channels, fastening a nerve to each of the second threads and pulling each nerve into the channel by means of said second threads. Said second threads are preferably substantially thinner than the first flexible structures. It may be possible to use the first flexible structures to pull nerve grafts into the channels. Alternatively, the step of inserting nerves and/or biological material promoting nerve regeneration in the channels comprises the step of pulling or pushing nerve grafts into the channels.

The peripheral nerves are preferably taken from another part of the patient's body.

The flexible elongate structures preferably have a cross-sectional dimension corresponding to the thickness of a nerve, so that the channels produced will also have a cross-sectional dimension corresponding to the thickness of a nerve.

Also disclosed are a set of moulds comprising a number of moulds according to the above. In this way a number of prefabricated moulds can be provided and the one that best matches an individual injury to be treated can be selected to produce the nerve regeneration device for this particular injury.

The nerve regeneration device may be made in any material that is biocompatible. A dental cement is proposed. The use of a biodegradable material such as fibrin (Tisel product) or modifications of this product with higher concentration of fibrinogen clotting to fibrin giving a more compact structure is useful. A biodegradable material is advantageous, since this will disappear in vivo, leaving only the regenerated axons. It will also provide a vehicle for sustained release of factors stimulating nerve regeneration. The nerve regeneration device may contain one or more of the following: nerve regeneration promoting factors such as aFGF, NFG, other growth factors, chABC and/or antibodies that will neutralize the action of nerve growth inhibiting components in the spinal cord and nerve roots. The material may be precharged with a growth factor, or a combination of growth factors, such as aFGF or chABC, and/or antibodies. Antibodies will neutralize the action of components in the spinal cord and roots that inhibit nerve regeneration. The growth factor and/or antibodies will then be gradually transferred from the nerve regeneration device to the nerve grafts, thus stimulating nerve regeneration over a period of time.

### Brief Description of the Drawings

The invention will be described in more detail in the following, by way of example and with reference to the appended drawings in which:
Figure 1 is a perspective view of a nerve regeneration device, and the ends of the central nervous system at a gap, where the pathways in the central nervous system are redirected.
Figure 2 is a flow chart of the inventive method.
Figure 3 illustrates how matching holes in the proximal and distal side wall can be positioned.
Figure 4 illustrates the proximal and distal side walls of a mould, with threads provided between some of the entry holes.
Figure 5 illustrates a mould held by a holder.
Figure 6 illustrates the mould according to figure 5 in a first and a second position.
Figure 7 illustrates the mould according to figure 5 in the second position with part of the wall removed.
Figure 8 shows an example of a resulting nerve regeneration device.

### Detailed Description of Embodiments

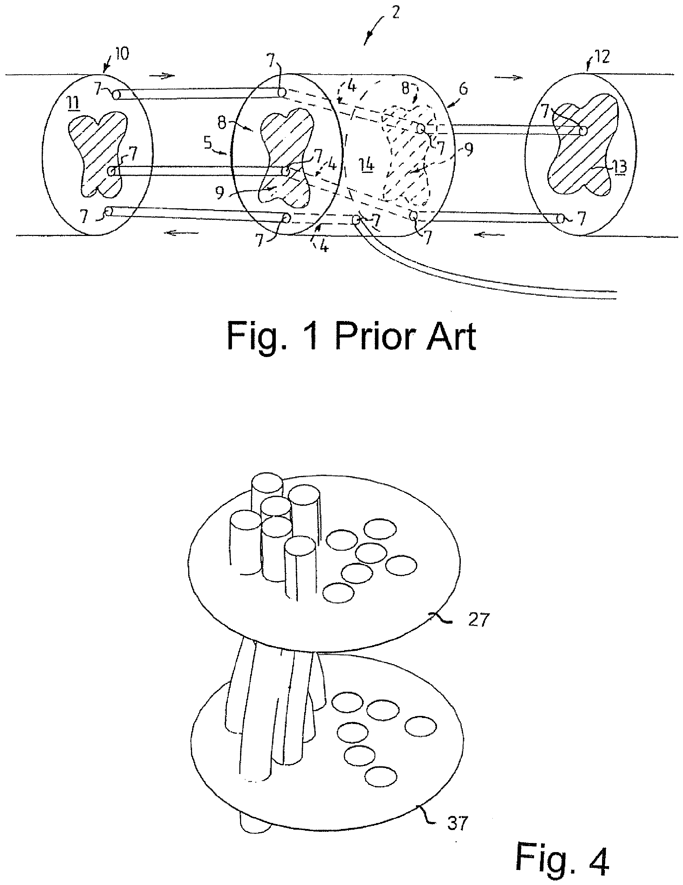

Figure 1 shows a nerve regeneration device 2 known in the art for use in the treatment of spinal cord injuries device.

The nerve regeneration device 2 has the form of a cylinder with a proximal end area 5 and a distal end area 6, each end area having first 8 and second 9 parts. The first parts 8 and the second parts 9 of the proximal and distal ends 5, 6 respectively, are white and shaded, respectively. The device contains channels 4, of which three are shown in the drawing. One channel 4 leads from the first part 8 of the proximal end area 5 to the second part 9 of the distal area 6. Another channel leads from the second part 9 of the proximal end area 5 to the first part 8 of the distal end area 6. One of the channels 4 ends in an opening 7 on the side area 14 of the device in order to be shunted past the distal end of the injury and introduced into the spinal cord further down at a suitable angle through the white into the grey matter. The channels have been threaded with peripheral nerve grafts (not shown). In the device of Figure 1 descending motor pathways from proximal white were coupled to distal grey matter and ascending pathways from distal white to proximal grey matter, according to the arrows. Instead of peripheral nerve grafts a biological material that promotes nerve regeneration may be inserted into the channels. Such biological material can be produced in vitro from stem cells or cells that are found around nerve fibres such as, for example, neurons, Schwann cells, macrophages such as microglia, and fibroblasts. Other examples of biological material that can be used in this context include extracellular matrix component structures, such as collagene-like structures, elastine, glucosaminoglucanes or other connective tissue substances. This applies throughout this document where nerve grafts are inserted into, or present in, the channels.

Figure 1 shows a nerve regeneration device for bridging a gap across the whole cross section of the spinal cord. According to the invention, the nerve regeneration device may be adapted to any shape and size of the injury covering a partial or full cross section of the spinal cord as well as any level of the spinal cord, all levels with unique dimensions, The end areas may be plane or have any other desired shape to match the end areas of the injury.

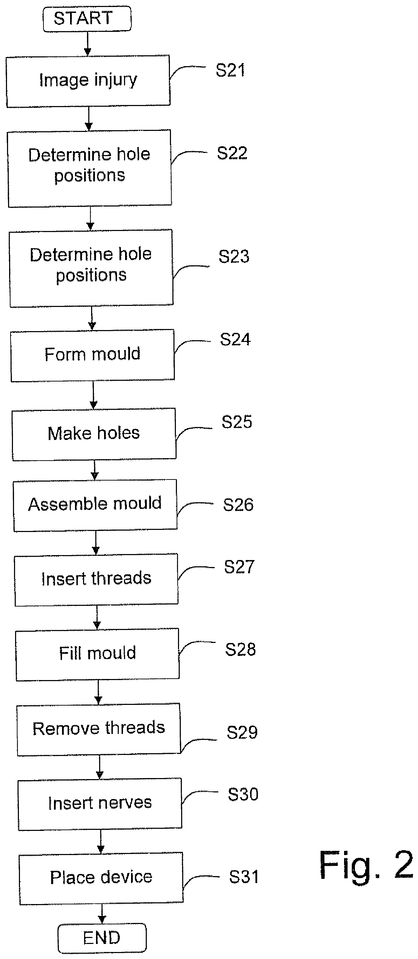

Figure 2 is a flow chart of the inventive method for producing the nerve regeneration device according to the invention. As will be obvious to the skilled person, some steps could change order, and/or be performed jointly, for example step S24 might be performed before step S23, or after step S25.

In step S21 the damaged area of the spinal cord is reproduced using a radiological imaging technique common in the art, to produce image data relating to the size and shape of the damaged area and the cross-section of the spinal cord at the position of the damage. Such imaging techniques include computer tomography, nuclear magnetic resonance and others, any of which may be used in the context of the present invention.

In step S22 the data obtained in step S21 are used to determine the shape and size of the nerve regeneration device that should be produced to bridge the proximal and distal ends of the injured area.

In step S23 the positions of the entry holes of each channel at each end of the nerve regeneration device are determined.

In step S24 the different parts of the mould in which the nerve regeneration device should be made are formed using any suitable method known in the art, such as turning or milling.

This includes forming the side walls of the mould, which may be one closed wall having a cross section that is essentially a full or partial circular or elliptic shape. The edges at both ends of the side walls may be even, or may have any suitable shape to match the injury. Also, the proximal and distal end walls are formed, having essentially the same shape as the cross-section of the side walls. The end walls may be plane or may have a suitable shape to match the edges of the side walls.

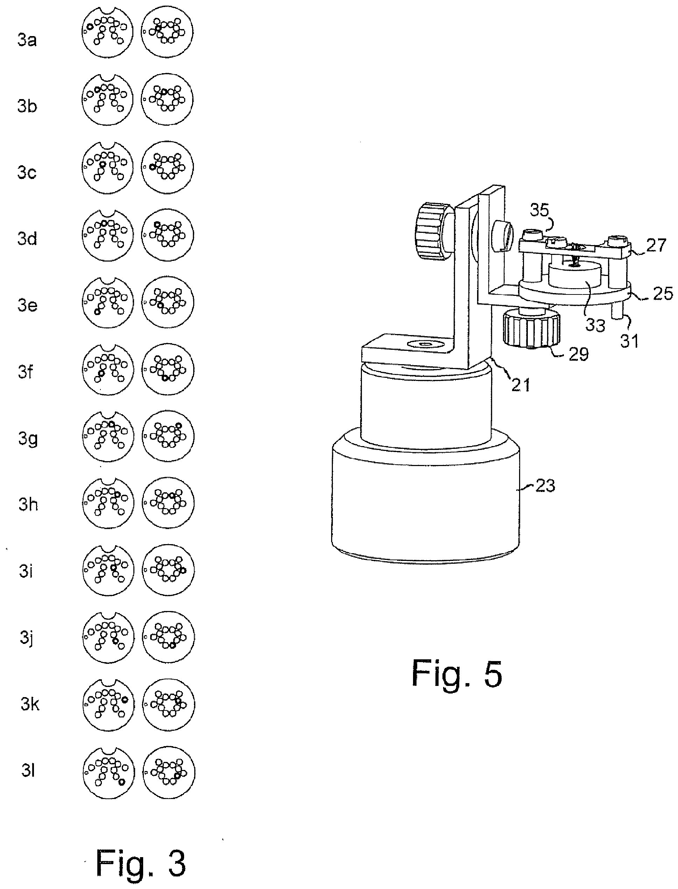

In step S25 holes are provided in the end walls, where entry points should be present for the nerves that are to form the connections between the proximal and distal ends. The positioning of the holes is discussed below, in connection with Fig. 3.

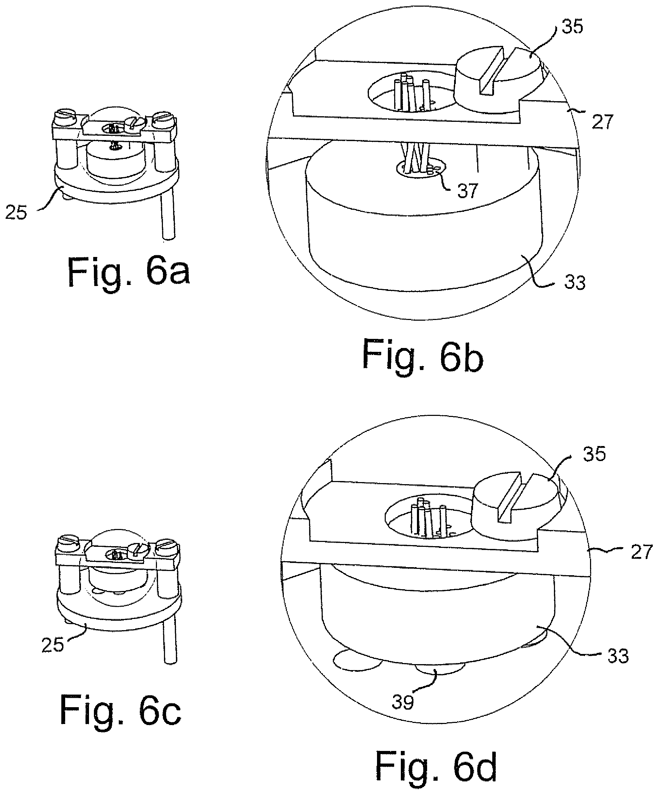

In step S26 the mould is partly assembled, for example, as discussed in connection with Figure 6, to allow threads to be placed in the mould between the entry points.

In step S27 threads, wires or other flexible elongate structures having a suitable cross-sectional dimension are placed between the entry points so that channels will be formed where the threads are running. For simplicity, the structures used are referred to as threads in this description. The threads may be nylon threads, for example, fishing line of a suitable dimension. An example of how to match the entry holes at the proximal and distal ends correctly is discussed in connection with Figure 3.

In step S28 the mould is filled with a suitable biocompatible or biodegradable material in which the nerve regeneration device is to be made. Suitable materials are discussed above. As stated above, the material may also be treated or mixed in various ways with growth factors or antibodies to promote regeneration of the nerves. The nerve regeneration device is then allowed to polymerize or solidify until it is dry.

In step S29 the threads are removed from the mould, resulting in open channels through the nerve regeneration device, which, when placed in the injured area, will connect the proximal and the distal ends in the desired points.

In step S30 the channels are filled with peripheral nerves taken from another part of the patient's body. This may be done by inserting a thin thread, much thinner than the thread used in step S27 to form the channels, through each channel, fastening the thin thread to a nerve, for example by tying them together, and pulling the nerve through the channel. Alternatively the nerve grafts may be sucked into the channels by means of a suction technique or inserted by a pushing technique involving pressurized gas or any other suitable method. A preloaded flexible tube comprising the nerve grafts may be pushed into the channel.

In step S31, when all the channels in the nerve regeneration device have been filled with nerve grafts, the nerve regeneration device is ready to be placed in the injured part of the spinal cord.

Instead of creating the mould to correspond to the damaged area, the mould can be made to create a larger device, which can then be shaped by cutting, milling or another suitable process to the desired size and shape. For example, in the case of a partial injury to the spinal cord, a device corresponding to the entire cross-section of the spinal cord can be made and the part of the device corresponding to the part of the spinal cord that is intact can be removed.

The steps related to imaging, determining the shape and size, and forming the mould are preferably performed by an imaging system as discussed above connected to a CAD/CAM system (Computer Aided Design/Computer Aided Manufacturing).

The threads used in step S27, as well as the holes in the top and bottom plates preferably have approximately the same dimensions as the nerves that are to be inserted in the channels.

Figure 3 illustrates how matching holes in the proximal and distal end plates can be positioned. Figure 3 is prepared for rats, but similar topographical maps can be prepared for human beings. The same proximal and distal end plates of a mould are shown in a number of pairs 3a - 31 of map. For each pair, the left part of the map shows the proximal end plate and the right part of the map shows the distal end plate. A dent is provided in the one end plate to enable filling the mould and for marking the direction. One pair of matching holes is marked in black for each pair of maps That is, the holes marked as black in the left and right part of the pair 3a are one pair of matching holes, between which a thread should be provided, to form a channel. As mentioned above, the end plates do not have to be elliptic, but can be part of a circle of ellipse as well.

The actual position of the holes will depend on the position of the nerve paths that are to be regenerated, which in turn depends on the position of the injury in the spinal cord.

In Figure 3 12 holes are shown, which has been found to be suitable in rats. The number of holes is restricted by the cross-sectional area and the shape of the device. For humans, having a thicker spinal cord, a greater number of holes will be feasible, for example 18 or 24 or an even greater number of holes. The greater the number of holes the more nerve paths can be regenerated, which will probably produce a better result. The holes must be placed in a way that is suitable for regenerating nerve paths in a human spinal cord. Maps of the human spinal cord can be found in a number of medical textbooks, for example, The human nervous system (2004) Paxinos & Mai, Academic Press Inc.

Figure 4 illustrates the proximal and distal end plates, or top 27 and bottom 37 plates, of a mould, with threads provided between some of the entry holes. For clarity, the side wall is not shown. As can be seen, the threads extend in curved lines to provide connections from white to grey matter.

Figure 5 illustrates a mould arranged in a holder 21 according to an embodiment of the invention. In this embodiment the mould is made of a suitable metal, but it may, of course, have any suitable design and be made from any other suitable material as well, such as plastic. The holder 1 may be shaped in any suitable way and is mounted on a base 23. As shown in Figure 5 the mould comprises a base plate 25, and a top plate 27, on which the end plates are provided. The base plate 25 and the top plate 27 are connected by means of two screws 29, 31, with distance elements between the plates 25, 27. Between the base plate 25 and the top plate 27 a centre part 33 is arranged. The top plate 27 and the base plate 25 define the shape of the two end walls of the nerve regeneration device. The centre part 33 has a through bore, which defines the length and cross-section of the nerve regeneration device The top plate 27 and the centre part 33 are connected by means of a third screw 35. The base plate 25 has a protrusion (not shown in Fig. 5), having the same cross section as the through bore of the centre part and comprising the holes for the proximal or distal end of the channels. The top plate 27 comprises the holes for the other end. By means of the third screw 35 the top plate 27 and the centre part 33 may be arranged in an open position, as shown in Figure 5, or in a closed position. In the open position the protrusion of the base plate extends substantially fully through the through bore of the centre part and in the closed position, only the end of the protrusion is located in the through bore. The open position will allow threads to be arranged between the holes in the top plate 27 and the base plate 25. In the closed position, the mould has the desired shape and may be filled with an appropriate material to manufacture the nerve regeneration device.

The top plate also comprises an entry hole (not shown) for introducing the biocompatible material into the mould. The mould may also be shaped so that a small protrusion is provided in the nerve regeneration device, for example, at the distal end, in the side facing inwards, to aid in placing the nerve regeneration device in the patient in the right way.

Of course, the mould can be provided in any suitable way, of which Figure 5 is only an example.

Figure 6 illustrates the open and closed positions of base plate 25, top plate 27 and centre part 33, as discussed in connection with Figure 5, more clearly. Figure 6a shows the open position, in which the end 37 of the protrusion 39 of the base plate 25 is visible at the end of the centre part 33 facing the top plate 27. The end 37 of the protrusion 39 constitutes the bottom plate of the mould. The threads can be seen extending upwards from the holes in the protrusion through the holes in the top plate 27. In Figure 6b the third screw 35 has been tightened, to raise the centre part 33 so that it meets the top plate 27. The protrusion 39 of the base plate 25 is visible on the base plate below the centre part 33. The threads can be seen extending up from the holes in the top plate 27. In this position the mould is ready for use.

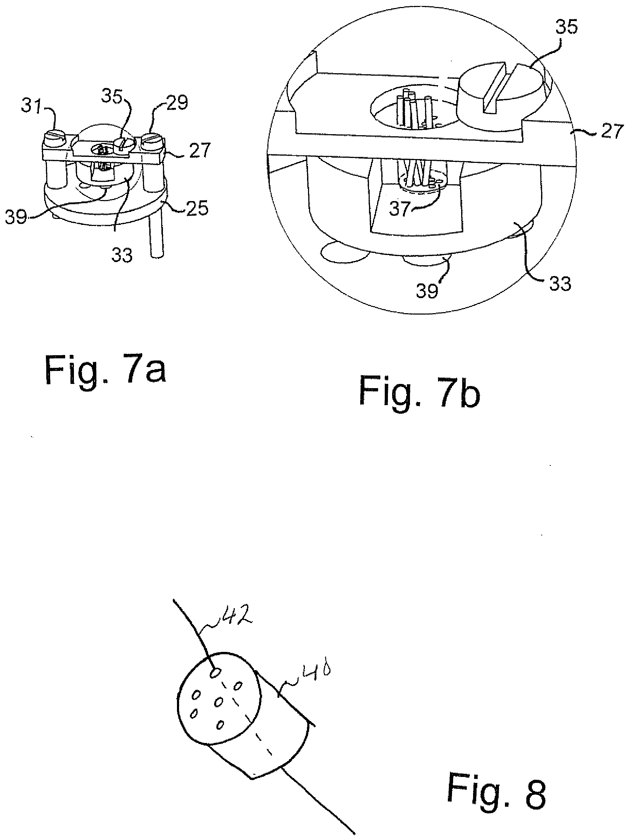

Figures 7a and 7b illustrate the bottom plate 25, the centre part 33 and the top plate 27 in the closed position, with part of the wall of the centre part removed to provide a view into the mould. As can be seen, the bottom plate 37 or end of the protrusion of the base plate 25 extends slightly into the through bore. The threads run from the holes in the protrusion 39, substantially along the axial direction of the through bore, and out through the holes in the top plate 27.

Figure 8 illustrates a nerve regeneration device 40 which may be produced according to an embodiment of the invention. In this example, the nerve regeneration device is arranged to bridge the entire cross-section of the spinal cord. A thread 42 is seen entering the nerve regeneration device 40 through a hole in the proximal end and exiting through the corresponding hole in the distal end of the nerve regeneration device.

The above description has primarily focused on connecting white matter to grey matter, because this is the method that is feasible today. It may be possible in the future to connect white matter to white matter, maybe even to regenerate one particular nerve path, especially with the development of exogeneously administered growth factors. This will potentially lead to a better function in the patient than the connection of white matter to grey matter. Of course, the inventive method and device can also be used to manufacture a nerve regeneration device for connecting white matter to white matter.

With the method and mould according to the invention a nerve regeneration device or treatment device can be manufactured to fit one particular injury in one particular patient, providing the best possible fit. Alternatively, it may be feasible to provide a number of standard sizes of devices, so that for each individual injury the best fit can be selected. Thus, a set of moulds having different dimensions may be provided for producing devices of different sizes.

## Claims

1. A method of manufacturing a nerve regeneration device (2) for treating a spinal cord injury of a patient, which may be a human or a vertebrate, said method comprising the following steps:
- imaging the spinal cord injury using an imaging technique, such as computer tomography or magnetic resonance imaging, obtaining image data related to the shape and size of the injury,
- providing a mould having a shape and size matching the spinal cord injury on the basis of said image data, said mould comprising a bottom plate (37), a top plate (27) and a centre part (33), each of said bottom plate (37) and said top plate (27) having a number of holes corresponding to points in the injured spinal cord where nerves should be regenerated, and said centre part having a through bore¹ therethrough,
- placing first flexible elongate structures in the through bore, each first flexible elongate structure exiting the mould through one hole in the top plate (27) and a matching hole in the bottom plate (37),
- filling the mould with a biocompatible or biodegradable material to produce the device,
- removing the device from the mould when ready, and
- removing the first flexible elongate structure from the device resulting in a first open channel through the nerve regeneration device (2)².

2. A method according to claim 1, further comprising the step of inserting nerves, and/or material promoting nerve regeneration, in said first open channel.

3. A method according to any one of the preceding claims, wherein the biocompatible or biodegradable material comprises an agent to stimulate nerve regeneration.
¹ Supported in WO2007/111562 on page 11, lines 23-24
² Supported in WO2007/111562 on page 9, lines 13-14 and previous claim 2.

4. A method according to any one of the claims 1-3, wherein each of the first flexible elongate structures is placed through holes in the top plates situated to correspond to grey matter area and holes in the base plates situated to correspond to white matter area, and vice versa.

5. A method according to any one of the preceding claims, wherein the step of inserting nerves in the channels comprises the steps of inserting threads in the channels, fastening a nerve to each of the threads and pulling each nerve into the channel by means of said threads.

6. A method according to any one of the claims 1- 4, wherein the step of inserting nerves and/or biological material promoting nerve regeneration in the channels comprises the step of pulling or pushing nerve grafts into the channels.

7. A method according to claim 5, wherein the said threads are substantially thinner than the first flexible elongate structures.

8. A method according to any one of the preceding claims, wherein the step of inserting nerves in the channels comprises inserting peripheral nerves taken from another part of the patient's body.

## Patentansprüche

1. Verfahren zur Herstellung einer Nervenregenerierungsvorrichtung (2) zur Behandlung einer Rückenmarksverletzung eines Patienten, bei welchem es sich um einen Menschen oder eine Wirbeltier handeln kann, wobei das Verfahren die folgenden Schritte umfasst:
- Bildgebung der Rückenmarksverletzung unter Verwendung einer Bildgebungstechnik, wie etwa Computertomographie oder Magnetresonanz-Bildgebung, wobei Bilddaten erhalten werden, die sich auf die Gestalt und die Größe der Verletzung beziehen,
- Bereitstellen, auf Grundlage der Bilddaten, einer Form, die eine Gestalt und Größe hat, welche der Rückenmarksverletzung entsprechen, wobei die Form eine untere Platte (37), eine obere Platte (27) und einen mittleren Abschnitt (33) umfasst, wobei die untere Platte (37) und die obere Platte (27) jeweils eine Anzahl von Löchern aufweisen, die Punkten in dem verletzten Rückenmark entsprechen, an welchen Nerven regeneriert werden sollen, und wobei durch den mittleren Abschnitt ein Durchgangsbohrung verläuft,
- Anordnen erster biegsamer länglicher Strukturen in der Durchgangsbohrung, wobei jede der biegsamen länglichen Strukturen die Form durch ein Loch in der oberen Platte (27) und ein entsprechendes Loch in der unteren Platte (37) verlässt,
- Füllen der Form mit einem bioverträglichen oder biologisch abbaubaren Material, um die Vorrichtung herzustellen,
- Entfernen der Vorrichtung aus der Form, wenn sie fertig ist, und
- Entfernen der ersten biegsamen länglichen Struktur aus der Vorrichtung, wodurch ein erster offener Kanal durch die Nervenregenerierungsvorrichtung (2) entsteht.

2. Verfahren gemäß Anspruch 1, wobei es weiterhin den Schritt des Einbringens von Nerven, und/oder von Material, welches die Nervenregenerierung fördert, in den ersten offenen Kanal umfasst.

3. Verfahren gemäß einem beliebigen der vorhergehenden Ansprüche, wobei das bioverträgliche oder biologisch abbaubare Material ein Mittel zum Stimulieren der Nervenregenerierung umfasst.

4. Verfahren gemäß einem beliebigen der Ansprüche 1 bis 3, wobei jede der ersten biegsamen länglichen Strukturen durch Löcher in den oberen Platten angeordnet wird, die derart gelegen sind, dass sie Gebieten mit grauer Substanz entsprechen, sowie Löcher in den unteren Platten, die derart gelegen sind, dass sie Gebieten mit weißer Substanz entsprechen, und umgekehrt.

5. Verfahren gemäß einem beliebigen der vorhergehenden Ansprüche, wobei der Schritt des Einbringens von Nerven in die Kanäle die Schritte des Einbringens von Fäden in die Kanäle, des Befestigens eines Nervs an jedem der Fäden sowie des Ziehens jedes der Nerven in den Kanal mittels der Fäden umfasst.

6. Verfahren gemäß einem beliebigen der Ansprüche 1 bis 4, wobei der Schritt des Einbringen von Nerven, und/oder von biologischem Material, welches die Nervenregenerierung fördert, in die Kanäle den Schritt des Ziehens oder Drücken von Nerventransplantaten in die Kanäle umfasst.

7. Verfahren gemäß Anspruch 5, wobei die Fäden im Wesentlichen dünner als die ersten biegsamen länglichen Strukturen sind.

8. Verfahren gemäß einem beliebigen der vorhergehenden Ansprüche, wobei der Schritt des Einbringens von Nerven in die Kanäle das Einbringen peripherer Nerven umfasst, welche aus einem anderen Teil des Körpers des Patienten entnommen wurden.

## Revendications

1. Procédé de fabrication d'un dispositif de régénération de nerf (2) pour traiter une blessure de la moelle épinière d'un patient, qui peut être un être humain ou un vertébré, ledit procédé comprenant les étapes suivantes :
- imagerie de la blessure de moelle épinière en utilisant une technique d'imagerie, telle que la tomographie informatique ou l'imagerie par résonance magnétique, obtenant des données d'images associées à la forme et à la taille de la blessure,
- fourniture d'un moule ayant une forme et une taille correspondant à la blessure de moelle épinière sur la base desdites données d'images, ledit moule comprenant une plaque de fond (37), une plaque de sommet (27) et une partie centrale (33), chacune de ladite plaque de fond (37) et de ladite plaque de sommet (27) ayant un nombre de trous correspondant aux points dans la moelle épinière blessée où les nerfs doivent être régénérés, et ladite partie centrale ayant une perforation à travers celle-ci,
- placement des premières structures allongées flexibles dans la perforation, chaque première structure allongée flexible sortant du moule à travers un trou dans la plaque de sommet (27) et un trou correspondant dans la plaque de fond (37),
- remplissage du moule avec un matériau biocompatible ou biodégradable pour produire le dispositif,
- enlèvement du dispositif du moule quand il est prêt, et
- enlèvement de la première structure allongée flexible du dispositif résultant à un premier canal ouvert à travers le dispositif de régénération de nerf (2).

2. Procédé selon la revendication 1, comprenant en outre l'étape d'insertion de nerfs, et/ou d'un matériau favorisant la régénération de nerf, dans ledit premier canal ouvert.

3. Procédé selon l'une quelconque des revendications précédentes, dans lequel le matériau biocompatible ou biodégradable comprend un agent pour stimuler la régénération de nerf.

4. Procédé selon l'une quelconque des revendications 1 à 3, dans lequel chacune des première structures allongées flexibles est placée à travers les trous dans les plaques de sommet situés pour correspondre à la zone de matière grise et les trous dans les plaques de base situés pour correspondre à la zone de matière blanche, et vice versa.

5. Procédé selon l'une quelconque des revendications précédentes, dans lequel l'étape d'insertion de nerfs dans les canaux comprend les étapes d'insertion de fils dans les canaux, l'attache d'un nerf à chacun des fils et le retrait de chaque nerf dans le canal au moyen desdits fils.

6. Procédé selon l'une quelconque des revendications 1 à 4, dans lequel l'étape d'insertion de nerfs et/ou de matériau biologique favorisant la régénération de nerf dans les canaux comprend l'étape de tirer ou de pousser des greffes de nerf dans les canaux.

7. Procédé selon la revendication 5, dans lequel lesdits fils sont substantiellement plus fins que les premières structures allongées flexibles.

8. Procédé selon l'une quelconque des revendications précédentes, dans lequel l'étape d'insertion de nerfs dans les canaux comprend l'insertion de nerfs périphériques prélevés d'une autre partie du corps du patient.
